# EUROPEAN PATENT SPECIFICATION

(11) **EP 3 366 251 B1**
(45) Date of publication and mention of the grant of the patent: **04.09.2019**
(21) Application number: 18167661.0
(22) Date of filing: 18.05.2016
(51) Int. Cl.: A61B 18/14, A61B 5/00, A61B 5/042

(54) **WOVEN FOLDABLE CATHETER**
GEWOBENER FALTBARER KATHETER
CATHÉTER PLIABLE TISSÉ

(30) Priority: 19.05.2015 US 201514715958
(43) Date of publication of application: 29.08.2018
(62) Divisional of application: 16170154.5
(73) Proprietor: Biosense Webster (Israel) Ltd., Yokneam 2066717 (IL)
(72) Inventor: BAR-TAL, Meir, 3224009 Haifa (IL); GARCIA, Ariel, Glendora, CA California 91741 (US); LOVEJOY, Erica Evelyenne, La Puente, CA California 91744 (US); HIGHSMITH, Debby Esther, Laguna Niguel, CA California 92677 (US); BOTZER, Lior, 3657600 Timrat (IL); HAIMOVICH, Roee, 3666004 Nesher (IL)
(74) Representative: Small, Gary James

(56) References cited:
- WO-A2-2015/187430
- US-A1- 2006 089 637
- US-A1- 2013 190 587
- US-B1- 6 514 249

## Description

### FIELD OF THE INVENTION

The present invention relates generally to catheters, and specifically to formation of the distal end of a catheter.

### BACKGROUND OF THE INVENTION

During a medical procedure on the heart, such as an ablation, the energy for the ablation may be radiofrequency energy that is injected into the heart via electrodes contacting the heart. The electrodes, or other electrodes, may also be used to monitor the condition of the heart, by acquiring signals from the heart as it beats. Present day ablation procedures typically use a relatively large number of electrodes simultaneously, and such electrodes may be provided in specially designed catheters, such as basket, pent-array or lasso catheters.

WO 2015/187430, published after the priority date of the present application and prior art under Article 54(3) EPC, discusses an expandable electrode assembly which includes multiple bipolar electrode pairs including a first electrode located on an outer surface and a second electrode located on an inner surface of the individual splines forming the expandable electrode assembly. Such an electrode arrangement may produce improved electrical activation signals which may be used to produce a more accurate map of the electrical activity of a patient's heart.

US 6,514,249 discusses a tissue ablation device assembly and method using a circumferential ablation member in combination with a position monitoring assembly in order to position the circumferential ablation member along a circumferential region of tissue at a location where a pulmonary vein extends from a left atrium.

US 2006/089637 discusses devices, systems and methods are for the ablation of tissue, including an ablation catheter which has an array of ablation elements attached to a deployable carrier assembly. The carrier assembly can be constrained within the lumen of a catheter, and deployed to take on an expanded condition.

US 2013/0190587 discusses a high-density electrode based medical device system, which may be achieved, at least in part, by overlapping elongate members on which the electrodes are located. The elongate members may be arranged in a structure which is moveable between an unexpanded configuration, which is suitable for percutaneous delivery through a bodily opening to a bodily cavity, and an expanded configuration, which is not.

### SUMMARY OF THE INVENTION

The present invention provides an apparatus, including:
a catheter having a distal end and a predefined outer diameter; and
a plurality of elastic filaments, each filament being formed from a conductive element, having at least one electrode fixed thereto and having two ends fixed within the catheter distal end to hold the filament as a loop. The loop intertwines with one or more other loops of the other filaments so that the plurality of the filaments forms an open lattice, which expands when uncompressed to a lattice diameter at least five times greater than the outer diameter of the catheter, wherein:
   the lattice diameter comprises a largest distance between selected sections of the plurality of elastic filaments;
   the open lattice when uncompressed comprises open spaces between the intertwined filaments ; and
      a ratio of a first total area defined by the open spaces and a second total area defined by the filaments is at least 5:1.

Typically, the open lattice compresses to fit within a cylinder having a diameter equal to the predefined outer diameter of the catheter.

In a disclosed embodiment the open lattice when uncompressed is sized has a spherical shape. Alternatively, the open lattice when uncompressed is an open glove or cone.

In a yet further disclosed embodiment each elastic filament consists of a tube having a lumen, and the at least one electrode is attached to a conductive wire traversing the lumen.

There is further provided, according to the present invention, a method, including:
providing a catheter with a distal end and a predefined outer diameter; and
fixing a plurality of elastic filaments within the catheter distal end, each filament being formed from a conductive element, having at least one electrode fixed thereto and having two ends fixed within the catheter distal end to hold the filament as a loop, wherein the loop intertwines with one or more other loops of the other filaments so that the plurality of the filaments forms an open lattice, and wherein the open lattice expands when uncompressed to a lattice diameter at least five times greater than the outer diameter of the catheter, wherein:
the lattice diameter comprises a largest distance between selected sections of the plurality of elastic filaments;
the open lattice when uncompressed comprises open spaces between the intertwined filaments; and
   a ratio of a first total area defined by the open spaces and a second total area defined by the filaments is at least 5:1.

The present disclosure will be more fully understood from the following detailed description of the embodiments thereof, taken together with the drawings, in which:

### BRIEF DESCRIPTION OF THE DRAWINGS

Fig. 1 is a schematic illustration of a minimally invasive medical system, according to an embodiment of the present invention;
Figs. 2, 3, 4, 5 and 6 are different schematic views of a distal end of a probe, according to embodiments of the present invention; and
Figs. 7 and 8 are two views illustrating an uncompressed open lattice, according to an alternative embodiment of the present invention.

### DETAILED DESCRIPTION OF EMBODIMENTS

### OVERVIEW

Many catheters, such as pent-array, lasso, or basket catheters, may push the heart wall when attempting to conform to the shape of the wall during a medical procedure, such as an ablation, performed on the wall. In addition, these types of catheters have relatively sharp regions (the ends of the splines in the case of a pent-array or lasso catheter; the distal end of the basket in the case of a basket catheter). For catheters with open ended constructions, such as the pent-array or lasso catheters, it is difficult to maintain their shape during operation. (In the pent-array catheter the ends tend to overlap; in the lasso catheter the lasso end tends to entangle with the remainder of the lasso, and relatively large forces are required to counteract these effects.) For all these types of catheters the combination of the required force and sharp regions may have undesired results during use of the catheters in medical procedures such as those referred to above.

Embodiments of the present invention overcome both of the problems. A plurality of elastic filaments are attached to the distal end of a catheter, each filament having fixed to it at least one electrode. The two ends of each filament are fixed within the catheter, typically within the distal tip of the distal end, so that each filament forms a loop with no sharp regions. The loops formed by the filaments are configured to intertwine in a woven manner with each other, so that the plurality of the filaments form an open lattice. The elasticity of the filaments allows the open lattice to exist in a compressed or in an uncompressed form.

In the compressed form, the catheter with its compressed open lattice may be inserted into a sheath that guides the distal end of the catheter to a desired location, typically in the heart during a procedure being performed on the heart. At the desired location the compressed open lattice exits the sheath, and decompresses to form an uncompressed open lattice.

The uncompressed open lattice is relatively large, having a lattice diameter at least five times larger than the outer diameter of the catheter distal end, so that the electrodes of the lattice are able to contact the heart wall. However, the elasticity of the filaments, and their lack of sharp edges, prevents trauma to the heart.

By having the loops of the filaments intertwine with, and cross, each other, in the woven manner referred to above, the uncompressed open lattice formed by the loops is able to maintain its shape.

### SYSTEM DESCRIPTION

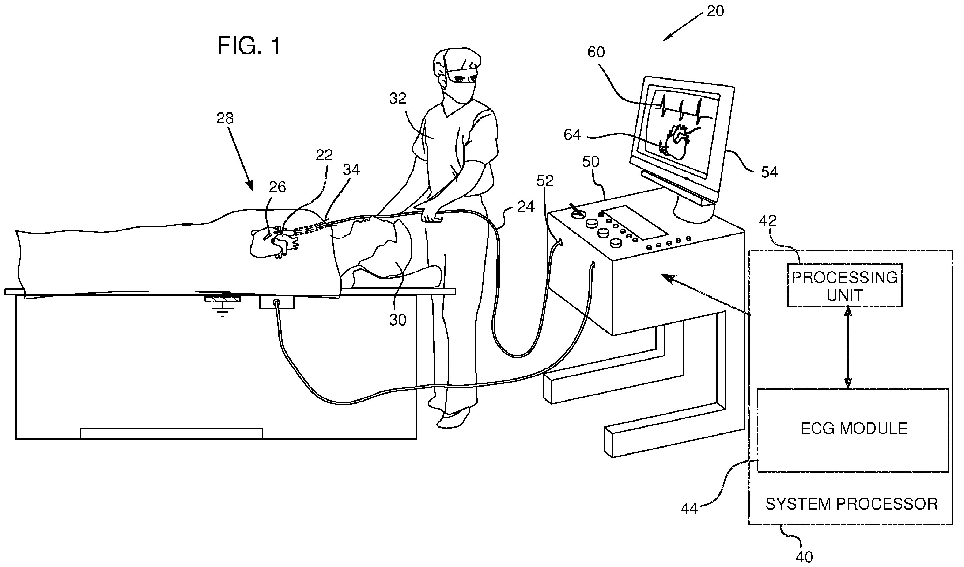

Reference is now made to Fig. 1, which is a schematic illustration of a minimally invasive medical system 20, according to an embodiment of the present invention. System 20 is typically used during a medical procedure on a body organ, and in the description herein the body organ, by way of example, is assumed to comprise the heart, wherein the system is applied to sample, and typically record and analyze, intra-cardiac electrocardiogram (ECG) signals. However, it will be understood that system 20 may be applied to sample other signals from other body organs.

The following description assumes that system 20 senses intra-cardiac ECG signals from a heart 22, using a probe 24. Probe 24 typically comprises a catheter, and is herein also referred to as catheter 24. A distal end 26 of the probe is inserted into the body of a subject 30. Distal end 26 of the probe, described in more detail below, comprises a plurality of electrodes 28 which sense the ECG signals. Prior to insertion of the probe into heart 22, a sheath 34 may be inserted into the subject until the distal end of the sheath is in a desired location. Once the distal end of the sheath has been correctly positioned, probe 24 may be inserted into sheath 34 until distal end 26 of the probe exits from the distal end of the sheath. In the description herein a user 32, typically a medical professional, is assumed to insert the sheath and the probe.

System 20 may be controlled by a system processor 40, comprising a processing unit 42 communicating with an ECG module 44. Processor 40 may be mounted in a console 50, which comprises operating controls which typically include a pointing device such as a mouse or trackball. Console 50 also connects to other elements of system 20, such as a proximal end 52 of catheter 24. Professional 32 uses the pointing device to interact with the processor, which, as described below, may be used to present results produced by system 20 to the professional on a screen 54.

The screen displays results of analysis and processing of ECG signals by ECG module 44. Typically, the resultant ECG signals are presented on screen 54 in the form of a potential vs. time graph, and a schematic example 60 of such a graph is illustrated in Fig. 1. However, the resultant ECG signals may also be used by processor 40 to derive other results associated with the ECG signals, such as a local activation time (LAT). These results are typically presented on screen 54 in the form of a three-dimensional (3D) map 64 of the internal surface of heart 22.

Processor 40 uses software stored in a memory of the processor to operate system 20. The software may be downloaded to processor 40 in electronic form, over a network, for example, or it may, alternatively or additionally, be provided and/or stored on non-transitory tangible media, such as magnetic, optical, or electronic memory.

Processor 40 typically comprises other modules, such as a probe tracking module and an ablation module that provides regulated power to one or more electrodes 28, or to one or more other electrodes in the distal end. For simplicity, such modules are not shown in Fig. 1. The Carto® system produced by Biosense Webster, of Diamond Bar, CA, uses such modules.

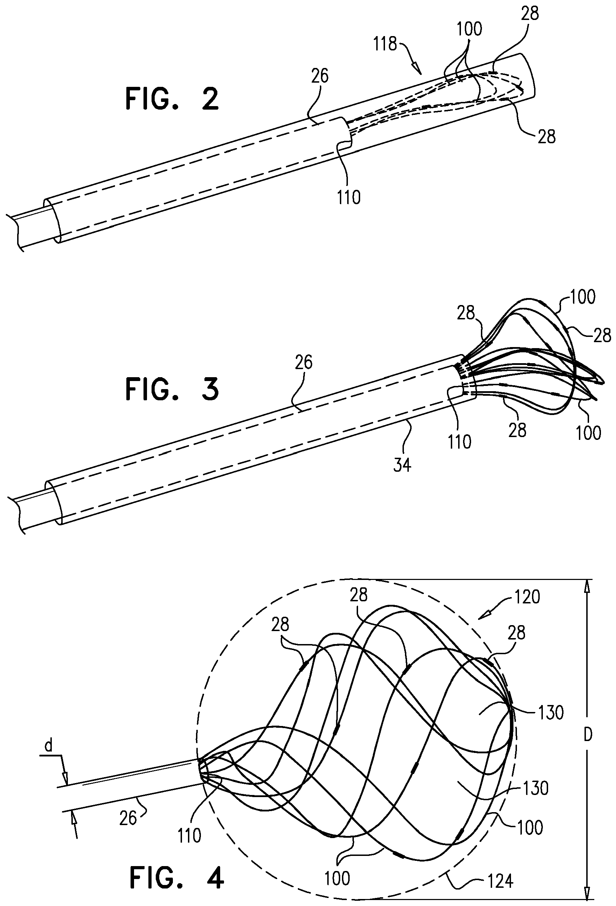

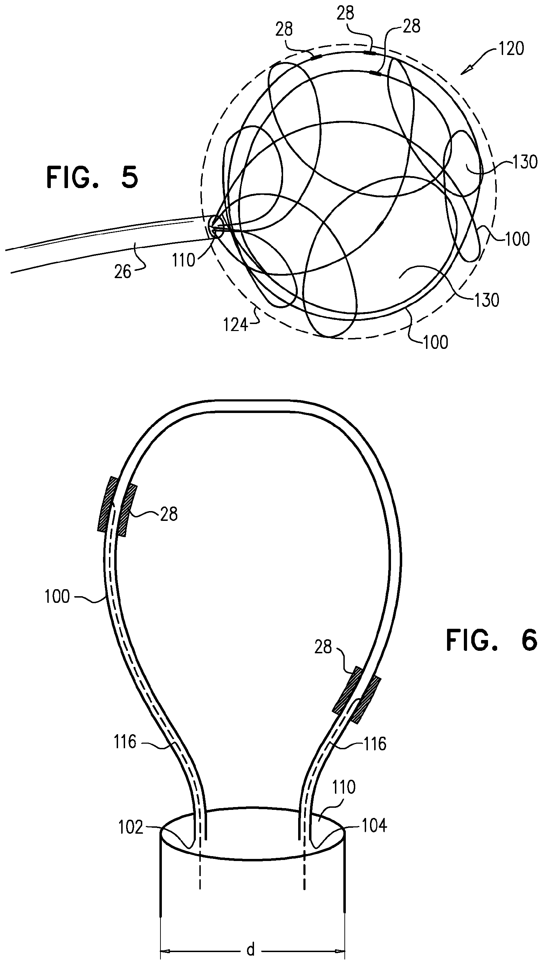

Figs. 2, 3, 4, 5 and 6 are different schematic views of distal end 26 of probe 24, according to embodiments of the present invention. Fig. 2 illustrates the probe distal end prior to exiting, or after reentering, the distal end of sheath 34. Fig. 3 illustrates probe distal end 26 when it is exiting or reentering sheath 34. Figs. 4 and 5 illustrate probe distal end 26 when it has completely exited sheath 34, in two different views. Fig. 6 schematically illustrates, in cross-section, a single loop attached to distal end 26.

Distal end 26 comprises a plurality of flexible elastic filaments 100, each filament 100 being typically formed from a conductive element such as a nitinol tube. As is illustrated in Fig. 6, each filament has two ends, 102, 104, both of which are fixed to distal end 26 of catheter 24, typically at a tip 110 of the distal end, so that each filament forms a loop. Distal end 26 of the catheter has an outer diameter d. If filaments 100 are conducting, for example if they are formed from nitinol, they are typically covered with an insulating material. Alternatively, filaments 100 may be formed from insulating material.

Each filament 100 has at least one electrode 28 fixed to the filament. If the filaments are in the form of a tube, conducting wires 116, insulated if filament 100 is conductive, may be attached through holes in the filaments to the electrodes, and the wires may be fed through and traverse a lumen of the tube (as illustrated in Fig. 6), via distal end 26 and proximal end 52 of catheter 24, to console 50. Signals acquired by the electrodes may thus be analyzed by processor 49. Alternatively, if filaments 100 are not tubular, wires 116 may be cemented to the outside of the filaments.

The filaments are fixed to distal end 26 so that the loops formed by each of the filaments intertwine to form an open lattice. The loops of the open lattice are arranged in a woven manner so that they interlace and cross each other, and so that they are able to slide against each other. Because of the elasticity of its constituent filaments, the open lattice may be in a compressed form as a compressed open lattice 118, illustrated in Fig. 2. The open lattice of the filaments may also be in an uncompressed form as an uncompressed open lattice 120. Two views of open lattice 120 are provided in Fig. 4 and Fig. 5. When the filaments are fixed to the distal end, they are arranged so that uncompressed open lattice 120 has a predefined shape, which is sized to fit within a virtual envelope. In the case of open lattice 120, it has a spherical shape and is sized so that it can be enclosed by a spherical virtual envelope 124.

Uncompressed open lattice 120 is formed of intertwined filaments 100, between which there are open spaces 130 (Figs. 4 and 5). In one embodiment, a ratio of a total area of the open spaces to a total area of filaments 100 is at least 20:1, where both areas are defined as the area produced by projection, from a center of the virtual envelope, of the open spaces and of the filaments onto the envelope. In other embodiments the ratio may be at least 5:1.

Uncompressed open lattice 120 also has a lattice diameter D, which is the largest distance between any two sections of filaments 100 forming the lattice. In some embodiments lattice diameter D may alternatively be considered as the largest distance between points on the virtual envelope sized to enclose the uncompressed open lattice, so that in the case of spherical virtual envelope 124, lattice diameter D corresponds to the diameter of envelope 124. Lattice diameter D is at least 5 times greater than outer diameter d of distal end 26 of the catheter, and is typically 20 times or more greater than d.

As stated above, in a typical cardiac procedure using system 20, sheath 34 is initially inserted so that the distal end of the sheath is in a desired location with respect to heart 22. Filaments 100 are compressed so that they form compressed open lattice 118, and so that the filaments are able to enter sheath 34. In some embodiments compressed open lattice 118 is small enough to fit into a cylinder having the same diameter d as the outer diameter of the distal end 26. In it's the lattice compressed form distal end 26 and its attached filaments may then be pushed into the sheath until it reaches the end of the sheath. Fig. 2 schematically illustrates distal end 26 with its attached filaments 100 within sheath 34, and Fig. 3 schematically illustrates the distal end and the filaments as the latter exit from the sheath.

After exiting from the sheath, filaments 100 decompress so that the filaments, in their uncompressed state, form uncompressed open lattice 120. At the termination of the procedure, distal end 26 may be pulled in a proximal direction, so that filaments 100 are compressed by the sheath and reenter the sheath as compressed open lattice 118.

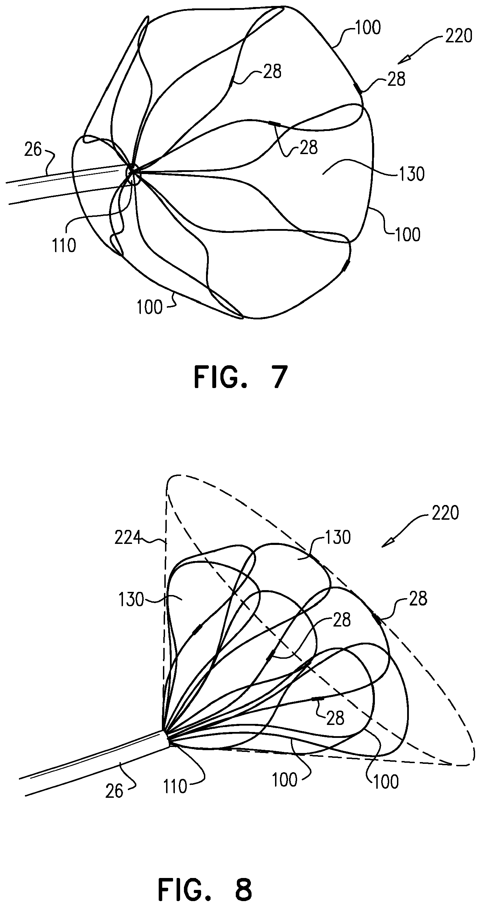

Figs. 7 and 8 are two views illustrating an uncompressed open lattice 220, according to an alternative embodiment of the present invention. Apart from the differences described below, uncompressed open lattice 220 is generally similar to uncompressed open lattice 120 (Figs. 4 and 5) and elements indicated by the same reference numerals in both lattices and in both sets of figures are generally similar in construction and in operation. Thus lattice 220 is formed from a plurality of intertwined filaments 100, each of the filaments having at least one electrode 28. Each of the filaments is fixed by respective ends of the filaments to distal tip 110, so that each filament is in the form of a loop. There are spaces 130 between intertwined filaments 100.

However, in contrast to uncompressed open lattice 120, the predefined shape of uncompressed open lattice 220 is an open glove or cone, so that lattice 220 is sized to be enclosed by a conical virtual envelope 224. As for lattice 120, lattice 220 has a lattice diameter D that is equal to the largest distance between any two sections of filaments 100 forming lattice 220. Alternatively, the lattice diameter may be considered as the largest distance between points on envelope 224. As for lattice 120, filaments 100 of uncompressed open lattice 220 may be compressed to form a compressed open lattice which is able to enter sheath 34.

It will be understood that embodiments of the present invention comprise other shapes, apart from the specific shapes described above with reference to uncompressed open lattices 120 and 220. For example, other uncompressed open lattices, formed of filaments 100 fixed to distal tip 110, all of which lattices may compress to fit within sheath 24, are in the form of an ellipsoid or a paraboloid. All such open lattices are assumed to be comprised within the scope of the present invention.

It will thus be appreciated that the embodiments described above are cited by way of example, and that the present invention is not limited to what has been particularly shown and described hereinabove. Rather, the scope of the present invention is defined by the claims.

## Claims

1. Apparatus, comprising:
a catheter (24) having a distal end (26) and a predefined outer diameter (d); and
a plurality of elastic filaments (100), each filament being formed from a conductive element, having at least one electrode (28) fixed thereto, and having two ends (102, 104) fixed within the catheter distal end to hold the filament as a loop, which intertwines with one or more other loops of the other filaments so that the plurality of the filaments forms an open lattice (120, 220), which expands when uncompressed to a lattice diameter (D) at least five times greater than the outer diameter of the catheter, wherein:
the lattice diameter (D) comprises a largest distance between selected sections of the plurality of elastic filaments;
the open lattice (120, 220) when uncompressed comprises open spaces (130) between the intertwined filaments (100); and
a ratio of a first total area defined by the open spaces and a second total area defined by the filaments is at least 5:1.

2. The apparatus according to claim 1, wherein the open lattice (120, 220) compresses to fit within a cylinder (34) having a diameter equal to the predefined outer diameter of the catheter (d).

3. The apparatus according to claim 1, wherein the open lattice (120) when uncompressed has a spherical shape.

4. The apparatus according to claim 1, wherein the predefined shape of the open lattice (220) when uncompressed is an open glove or cone.

5. The apparatus according to claim 1, wherein each elastic filament (100) comprises a tube having a lumen, and wherein the at least one electrode (28) is attached to a conductive wire (116) traversing the lumen.

6. The apparatus according to claim 1, wherein the conductive element is a nitinol tube.

7. The apparatus according to claim 1 or claim 6, wherein each filament is covered with an insulating material.

8. A method, comprising:
providing a catheter (24) with a distal end (26) and a predefined outer diameter (d); and
fixing a plurality of elastic filaments (100) within the catheter distal end, each filament being formed from a conductive element, having at least one electrode (28) fixed thereto, and having two ends (102, 104) fixed within the catheter distal end to hold the filament as a loop, wherein the loop intertwines with one or more other loops of the other filaments so that the plurality of the filaments forms an open lattice (120, 220), and wherein the open lattice expands when uncompressed to a lattice diameter (D) at least five times greater than the outer diameter of the catheter, wherein:
the lattice diameter (D) comprises a largest distance between selected sections of the plurality of elastic filaments;
the open lattice (120, 220) when uncompressed comprises open spaces (130) between the intertwined filaments (100); and
a ratio of a first total area defined by the open spaces and a second total area defined by the filaments is at least 5:1.

9. The method according to claim 8, wherein the open lattice (120, 220) compresses to fit within a cylinder (34) having a diameter equal to the predefined outer diameter of the catheter (d).

10. The method according to claim 8, wherein the open lattice (120) when uncompressed has a spherical shape.

11. The method according to claim 8, wherein the open lattice (220) when uncompressed is an open glove or cone.

12. The method according to claim 8, wherein each elastic filament (100) comprises a tube having a lumen, and wherein the at least one electrode (28) is attached to a conductive wire (116) traversing the lumen.

13. The method according to claim 8, wherein the conductive element is a nitinol tube.

14. The method according to claim 8 or claim 13, wherein each filament is covered with an insulating material.

## Patentansprüche

1. Vorrichtung, umfassend:
einen Katheter (24) mit einem distalen Ende (26) und einem vordefinierten Außendurchmesser (d); und
eine Vielzahl von elastischen Filamenten (100), wobei jedes Filament aus einem leitfähigen Element gebildet ist, wenigstens eine daran befestigte Elektrode (28) aufweist und zwei Enden (102, 104) aufweist, die innerhalb des distalen Endes des Katheters befestigt sind, um das Filament als Schleife zu halten, die mit einer oder mehreren anderen Schleifen der anderen Filamente verschlungen ist, so dass die Vielzahl der Filamente ein offenes Gitterwerk (120, 220) bildet, das, wenn unkomprimiert, auf einen Gitterdurchmesser (D) expandiert, der wenigstens fünfmal größer als der Außendurchmesser des Katheters ist, wobei:
der Gitterdurchmesser (D) einen größten Abstand zwischen ausgewählten Abschnitten der Vielzahl von elastischen Filamenten umfasst;
das offene Gitterwerk (120, 220), wenn unkomprimiert, offene Räume (130) zwischen den verschlungenen Filamenten (100) umfasst; und
das Verhältnis einer ersten Gesamtfläche, die von den offenen Räumen definiert ist, und einer zweiten Gesamtfläche, die von den Filamenten definiert ist, wenigstens 5:1 beträgt.

2. Vorrichtung gemäß Anspruch 1, wobei das offene Gitterwerk (120, 220) komprimiert, um in einen Zylinder (34) zu passen, der einen Durchmesser gleich dem vordefinierten Außendurchmesser des Katheters (d) aufweist.

3. Vorrichtung gemäß Anspruch 1, wobei das offene Gitterwerk (120), wenn unkomprimiert, eine sphärische Form aufweist.

4. Vorrichtung gemäß Anspruch 1, wobei die vordefinierte Form des offenen Gitterwerks (220), wenn unkomprimiert, ein offener Handschuh oder Kegel ist.

5. Vorrichtung gemäß Anspruch 1, wobei jedes elastische Filament (100) ein Rohr mit einem Lumen umfasst und wobei die wenigstens eine Elektrode (28) an einem leitfähigen Draht (116), der das Lumen durchläuft, befestigt ist.

6. Vorrichtung gemäß Anspruch 1, wobei das leitfähige Element ein Nitinol-Rohr ist.

7. Vorrichtung gemäß Anspruch 1 oder Anspruch 6, wobei jedes Filament mit einem Isoliermaterial überzogen ist.

8. Verfahren, umfassend:
Bereitstellen eines Katheters (24) mit einem distalen Ende (26) und einem vordefinierten Außendurchmesser (d); und
Befestigen einer Vielzahl von elastischen Filamenten (100) innerhalb des distalen Endes des Katheters, wobei jedes Filament aus einem leitfähigen Element gebildet ist, wenigstens eine daran befestigte Elektrode (28) aufweist und zwei Enden (102, 104) aufweist, die innerhalb des distalen Endes des Katheters befestigt sind, um das Filament als Schleife zu halten, wobei die Schleife mit einer oder mehreren anderen Schleifen der anderen Filamente verschlungen ist, so dass die Vielzahl der Filamente ein offenes Gitterwerk (120, 220) bildet, und wobei das offene Gitterwerk, wenn unkomprimiert, auf einen Gitterdurchmesser (D) expandiert, der wenigstens fünfmal größer als der Außendurchmesser des Katheters ist, wobei:
der Gitterdurchmesser (D) einen größten Abstand zwischen ausgewählten Abschnitten der Vielzahl von elastischen Filamenten umfasst;
das offene Gitterwerk (120, 220), wenn unkomprimiert, offene Räume (130) zwischen den verschlungenen Filamenten (100) umfasst; und
das Verhältnis einer ersten Gesamtfläche, die von den offenen Räumen definiert ist, und einer zweiten Gesamtfläche, die von den Filamenten definiert ist, wenigstens 5:1 beträgt.

9. Verfahren gemäß Anspruch 8, wobei das offene Gitterwerk (120, 220) komprimiert, um in einen Zylinder (34) zu passen, der einen Durchmesser gleich dem vordefinierten Außendurchmesser des Katheters (d) aufweist.

10. Verfahren gemäß Anspruch 8, wobei das offene Gitterwerk (120), wenn unkomprimiert, eine sphärische Form aufweist.

11. Verfahren gemäß Anspruch 8, wobei das offene Gitterwerk (220), wenn unkomprimiert, ein offener Handschuh oder Kegel ist.

12. Verfahren gemäß Anspruch 8, wobei jedes elastische Filament (100) ein Rohr mit einem Lumen umfasst und wobei die wenigstens eine Elektrode (28) an einem leitfähigen Draht (116), der das Lumen durchläuft, befestigt ist.

13. Verfahren gemäß Anspruch 8, wobei das leitfähige Element ein Nitiniol-Rohr ist.

14. Verfahren gemäß Anspruch 8 oder Anspruch 13, wobei jedes Filament mit einem Isoliermatieral überzogen ist.

## Revendications

1. Appareil comprenant :
un cathéter (24) ayant une extrémité distale (26) et un diamètre externe prédéfini (d) ; et
une pluralité de filaments élastiques (100), chaque filament étant formé à partir d'un un élément conducteur, ayant au moins une électrode (28) fixée à celui-ci, et ayant deux extrémités (102, 104) fixées à l'intérieur de l'extrémité distale du cathéter pour maintenir le filament sous la forme d'une boucle, qui s'entrelace avec une ou plusieurs autres boucles des autres filaments de sorte que la pluralité des filaments forme un treillis ouvert (120, 220), qui se déploie lorsqu'il est non comprimé à un diamètre de treillis (D) au moins cinq fois supérieur au diamètre externe du cathéter,
le diamètre de treillis (D) comprenant une distance la plus grande entre des sections sélectionnées de la pluralité de filaments élastiques ;
le treillis ouvert (120, 220) lorsqu'il est non comprimé comprenant des espaces ouverts (130) entre les filaments entrelacés (100) ; et
un rapport d'une première surface totale définie par les espaces ouverts et une seconde surface totale définie par les filaments étant d'au moins 5:1.

2. Appareil selon la revendication 1, le treillis ouvert (120, 220) se comprimant pour s'adapter à l'intérieur d'un cylindre (34) ayant un diamètre égal au diamètre externe prédéfini du cathéter (d).

3. Appareil selon la revendication 1, le treillis ouvert (120), lorsqu'il est non comprimé, ayant une forme sphérique.

4. Appareil selon la revendication 1, la forme prédéfinie du treillis ouvert (220) lorsqu'il est non comprimé étant un gant ou un cône ouvert.

5. Appareil selon la revendication 1, chaque filament élastique (100) comprenant un tube ayant une lumière, et la ou les électrodes (28) étant fixées à un fil conducteur (116) traversant la lumière.

6. Appareil sleon la revendication 1, l'élément conducteur étant un tube en nitinol.

7. Appareil selon la revendication 1 ou la revendication 6, chaque filament étant recouvert d'un matériau isolant.

8. Procédé comprenant :
la fourniture d'un cathéter (24) avec une extrémité distale (26) et un diamètre externe prédéfini (d) ; et
la fixation d'une pluralité de filaments élastiques (100) à l'intérieur de l'extrémité distale du cathéter, chaque filament étant formé à partir d'un élément conducteur, ayant au moins une électrode (28) fixée à celui-ci, et ayant deux extrémités (102, 104) fixées à l'intérieur de l'extrémité distale du cathéter pour maintenir le filament sous la forme d'une boucle, la boucle s'entrelaçant avec une ou plusieurs autres boucles des autres filaments de sorte que la pluralité des filaments forme un treillis ouvert (120, 220), et le treillis ouvert se déployant lorsqu'il est non comprimé à un diamètre de treillis (D) au moins cinq fois supérieur au diamètre externe du cathéter,
le diamètre de treillis (D) comprenant une plus grande distance entre des sections sélectionnées de la pluralité de filaments élastiques ;
le treillis ouvert (120, 220), lorsqu'il est non comprimé, comprenant des espaces ouverts (130) entre les filaments entrelacés (100) ; et
un rapport d'une première surface totale définie par les espaces ouverts et une seconde surface totale définie par les filaments étant d'au moins 5:1.

9. Procédé selon la revendication 8, le treillis ouvert (120, 220) se comprimant pour s'adapter à l'intérieur d'un cylindre (34) ayant un diamètre égal au diamètre externe prédéfini du cathéter (d).

10. Procédé selon la revendication 8, le treillis ouvert (120), lorsqu'il est non comprimé, ayant une forme sphérique.

11. Procédé selon la revendication 8, le treillis ouvert (220), lorsqu'il est non comprimé, étant un gant ou un cône ouvert.

12. Procédé selon la revendication 8, chaque filament élastique (100) comprenant un tube ayant une lumière, et la ou les électrodes (28) étant fixées à un fil conducteur (116) traversant la lumière.

13. Procédé selon la revendication 8, l'élément conducteur étant un tube en nitinol.

14. Procédé selon la revendication 8 ou la revendication 13, chaque filament étant recouvert d'un matériau isolant.
